# EUROPEAN PATENT APPLICATION

(11) **EP 4 527 317 A1**
(43) Date of publication of application: **26.03.2025**
(21) Application number: 24201012.2
(22) Date of filing: 18.09.2024
(51) Int. Cl.: A61B 17/00, A61F 5/445

(54) **DEVICE FOR REPOSITIONING OF A STOMA PROLAPSE**

(30) Priority: 19.09.2023 DK PA202330219
(71) Applicant: T Hove Holding ApS, 2820 Gentofte (DK)
(72) Inventor: HOVE, Thomas, 2820 Gentofte (DK)
(74) Representative: Larsen & Birkeholm A/S

(57) **Abstract**

The present invention relates to device or tool for repositioning a stoma prolapse. The present invention also relates to an assembly comprising a device or tool for repositioning a stoma prolapse combined with a positioning unit preventing a stoma prolapse from occurring.

In particular, the invention relates to a device for repositioning a stoma prolapse, comprising
- a container (1) comprising side walls (2), a first end (4) and a second end (5) wherein the second end (5) comprises an opening,
- a piston (3) dimensioned to travel inside the container (1) from the first end (4) of the container (1) to the second end (5), and
- a push device (6) configured to move the piston (3) from a first position to a second position close to or in contact with the second end (5).

## Description

The present invention relates to device or tool for repositioning a stoma prolapse. The present invention also relates to an assembly comprising a device or tool for repositioning a stoma prolapse combined with a positioning unit preventing a prolapse from occurring.

### Background of the invention

Various kinds of infirmaries of the human bowel can result in a surgical intervention in which the intestine is cut, and one end is brought out through an artificial opening in the abdominal wall called a stoma. Such stoma surgery thereafter requires the use of a replaceable ostomy bag to collect faecal matter otherwise passing normally from the colon.

Once the surgery around the stoma has healed, too large of a surgical opening in the abdominal wall, chronic coughing, obesity, ordinary peristalsis of the bowel, and stool passing can cause pressures inside the abdomen to development around the stoma to stretch the tissues and progressively prolapse the stoma through the abdominal wall. The severity of prolapse can vary from a relatively small 2-3 centimetres to a large 10+ centimetre prolapse.

If not too severe, a prolapse can be managed without resorting to more surgery to address the prolapse. Medically trained personal may manually push the prolapse back into the correct position behind the abdominal wall, however, the procedure may be difficult and long lasting.

Chinese utility model CN215839837 U discloses a suppository structure for treating stoma prolapse. The device comprises a prolapse suppository body, one end of the prolapse suppository body is of a T-shaped structure, the prolapse suppository body comprises a main body and a handle wing, the main body is of a hollow circular truncated cone structure, and the handle wing and the end of the main body are integrally arranged. The end, away from the handle wing, of the body is of an arc-shaped structure, the end of the arc-shaped structure enables the prolapse suppository body to be inserted into the intestinal canal more smoothly, the outer wall of the handle wing is of an arc-shaped structure, a mounting groove is formed in the outer wall of the handle wing, a limiting pad is mounted in the mounting groove, and the side, close to the body, of the limiting pad is flush with the side face of the handle wing. The prolapse suppository body is prevented from moving towards the interior of the intestinal canal, and a plurality of sealing rings of annular structures are integrally arranged on the side, close to the main body, of the limiting pad. The device is not a tool for repositioning a stoma-prolapse, it is a tool for preventing a stoma-prolapse.

Hence, a tool or a device for repositioning the prolapse behind the abdominal wall is in high demand, especially a tool or device which does not require assistance from medically trained personal, but which may be handled by the stoma-owner or a relative without general medical training.

### Summary of the invention

Thus, an object of the present invention relates to providing a device which reduces the time spend on repositioning prolapsed stomas as well as making it possible for a patient to reposition the patient's own prolapsed stoma.

Thus, one aspect of the invention relates to a device for repositioning a stoma prolapse, which device comprises a container (1) comprising side walls (2), a first end (4) and a second end (5) wherein the second end (5) comprises an opening adapted to fit a prolapsed stoma, a piston (3) dimensioned to travel from the first end (4) of the container (1) to the second end (5) inside the container (1), a push device (6) configured to move the piston (3) from a first position to a second position close to or in contact with the second end (5).

According to an embodiment of the invention, the push device (6) may comprise a rigid connection (7a) extending from the piston (3) to a handle (8) positioned outside the container (1).

According to an embodiment of the invention, the push device (6) may comprise an opening (9) at the first end (4) of the container (1) which opening (9) via a pipe or conduit (7b) is connected to a pumping unit (10) pumping a fluid such as air.

According to an embodiment of the invention, the container (1) may comprise an opening (11) at or near the second end (5) of the container (1).

According to an embodiment of the invention, the container (1) may comprise at least two openings (11, 12) at or near the second end (5) of the container (1) where a first opening (11) may function as an inlet opening and a second opening (12) may function as an outlet opening.

According to an embodiment of the invention, the device may comprise a connecting surface (13) at the second end (5) configured to provide a liquid tight contact. Such a connecting surface 13 may ensure or improve fluid-tightness in such a way that a liquid cannot pass out of the container (1) during operation or only pass out of the container (1) during operation in limited or very small quantities

According to an embodiment of the invention, the sidewalls of the container (1) may constitute a tube having a round or oval or elliptic or polygonal cross section, and/or the cross section of the tube (2) may vary in the longitudinal direction e.g. as in a truncated cone.

According to an embodiment of the invention, the container (1) may be made of a rigid, preferably watertight, material such as a polymer, laminated cardboard, or the like, according to a preferred embodiment the material of the container (1) may also be transparent such as e.g. acrylic, pvc, polycarbonate or the like, also the material may be adapted for multiple use or for single use.

According to a second aspect of the invention, the invention relates to an assembly comprising a device according to one of the claims 1-8 and a positioning unit (14) to be positioned around the stoma, wherein the unit (14) may be placed inside the container (1) before and/or during operation.

According to an embodiment of the second aspect, the unit (14) may comprise an edge part (16), a vaulted central part (17) which vaulted central part (17) comprises an opening (18).

### Brief description of the figures

Figure 1 shows a first embodiment of a device for repositioning a stoma prolapse according to the invention.
Figure 2 shows a second embodiment of a device for repositioning a stoma prolapse according to the invention The present invention will now be described in more detail in the following.
Figure 3 shows an embodiment of a push device to be connected to the container of the invention.
Figure 4 shows a unit which in position around the stoma prevents a stoma prolapse.

### Detailed description of the invention

### Definitions

Prior to discussing the present invention in further details, the following terms and conventions will first be defined:
*In general* - when this expression is used when mentioning a feature relating to the present invention, it must be understood that the feature may be used with all embodiments of the invention, even if the mentioning is made in the detailed part of the document.

It should be noted that embodiments and features described in the context of one of the aspects of the present invention also apply to the other aspects of the invention.

The invention will now be described in further details in the following non-limiting examples.

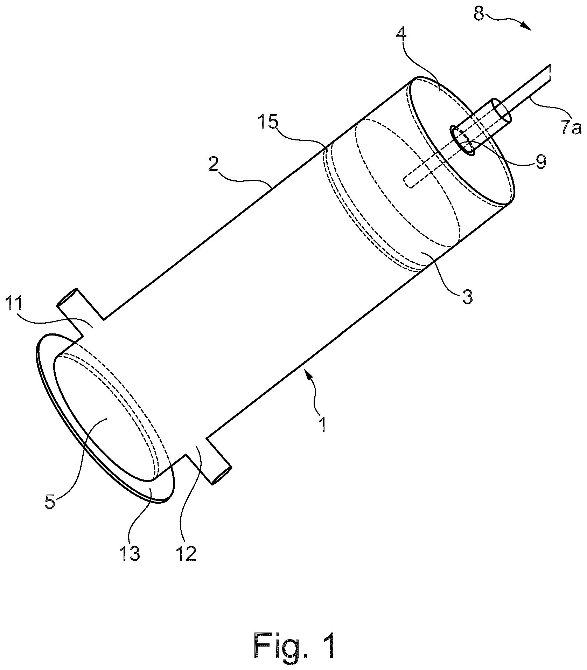

Fig. 1 shows a first embodiment of a device according to the invention. The first embodiment comprises a container 1 comprising side walls 2 which container 1 has a first end 4 connected to a push device 6 and a second end 5 which is open and allows for insertion of a prolapsed stoma.

In the first embodiment, the side walls 2 are constituted of a cylinder, however, the side walls 2 may have any shape and may in general be constituted of a tube having a round or oval or elliptic or polygonal cross section or the cross section of the tube may vary in the longitudinal direction e.g. decrease or increase as in a truncated cone.

In general, the size of the cross section and/or the size of the length of the container 1 or the tube constituting the side walls 2 of the container 1 may be adapted to a specific stoma prolapse. Adaptation of the size of the opening inside the container 1 will normally make it easier and faster to reposition the prolapse.

The first embodiment further comprises a piston 3. In general, the piston 3 may provide a fluid tight or almost fluid tight reduction of the volume between the surface of the piston 3 facing the second end 5 and the second end 5. During operation, the piston 3 is moved from a position close to or in contact with the first end 4, and to a position close to or in contact with the second end 5. The piston 3 may comprise a sealing ring 15 which assist in providing a fluid tight separation between a first volume defined by the side walls 2 of the container 1 between the piston 13 and the second end 5 and a second volume defined by the side walls 2 of the container 1 and the first end 4.

Only part of a push device 6 is shown in fig. 1, the parts shown in fig. 1 is a connection 7a which is connected to a not shown handle 8. The connection 7a may be a rigid stick or tube connected to a handle 8 and being of a length allowing the piston 3, the connection 7a and the handle 8 to travel from a first position close to or in contact with the first end 4 and to a second position close to the second end 5. A rigid connection 7a will allow the piston 3 to be pushed from the first position to the second position.

The container 1 is normally provided with an opening 9 through the first end 4 or close to the first end 4. The opening 9 will allow access for a connection 7a forcing the piston 3 towards the second end 5. However, there might be solutions without such an opening 9 e.g. if the surface of the first end 4 is displaceable into the volume of the container 1, then a user may displace the end surface into the container 1 e.g. by a finger or a tool.

In general, a device according to the invention may at or near the second end 5, comprise an opening 11 through the side walls 2 of the container 1. The opening 11 allows filling the container 1 with fluid during operation after having inserted the prolapsed stoma into the container 1.

In general, a device according to the invention may at or near the second end 5, comprise two or more openings 11, 12 for inlet and outlet of fluid. One or more inlets for fluid may be controlled by a pump and one or more outlets for fluid may be controlled by common or individual valve(s) e.g. opening when a certain pressure is built up inside the container 1.

The fluid may be a liquid or a gas providing a desired condition such as low temperature or the like for the prolapsed stoma during operation.

The first embodiment also comprises a connecting surface 13 which during operation is forced against the skin surface surrounding the prolapsed stoma. In general, the connecting surface 13 may comprise a sealing layer e.g. made of silicone or similar soft and compliant material.

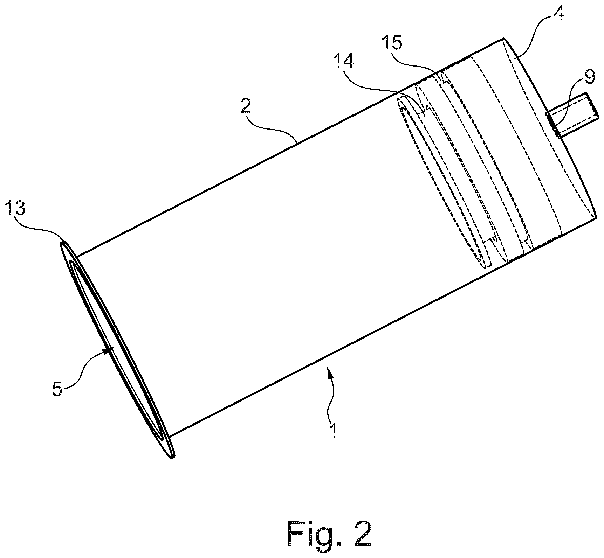

Fig. 2 shows a second embodiment. The second embodiment also comprises a cylindrical tube-shaped container 1 comprising a first end 4 with a small opening 9 through which a connection 7a is to be inserted, and a second end 5 with a large opening which large opening basically comprise the whole area of the second end 5 through which large opening a prolapsed stoma is to be inserted during operation.

Attached to the piston 3 of the second embodiment, is a positioning unit 14 which is temporarily attached to the piston 3 and via the piston 3 is to be brought into position on the abdominal wall around the stoma during operation.

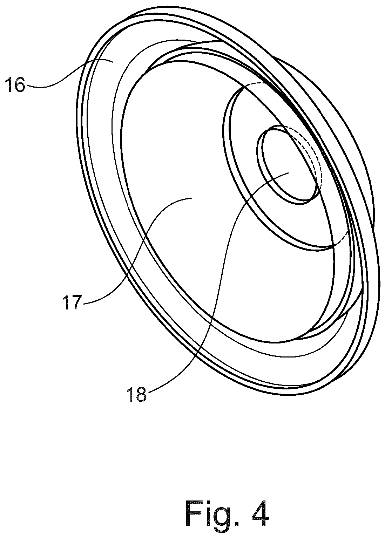

Fig. 4 shows an embodiment of such a positioning unit 14.

In general, a positioning unit 14 according to the invention may comprise an outer edge part 16 which is flat or slightly rounded and a vaulted central part 17 which vaulted central part 17 comprises an opening 18. The opening 18 of the vaulted central part 17 may be positioned centrally, but the position and/or the size of the opening 18 may also be adapted to the individual stoma. After mounting, the edge part 16 of the positioning unit 14 is to be positioned in contact with the outer surface of the abdominal wall around the stoma, and the positioning unit 14 may be kept in the mounted position by e.g. an adhesive or a belt or the like or it may be clicked or locked to a coupling adhered to the patient's abdominal surface around the stoma.

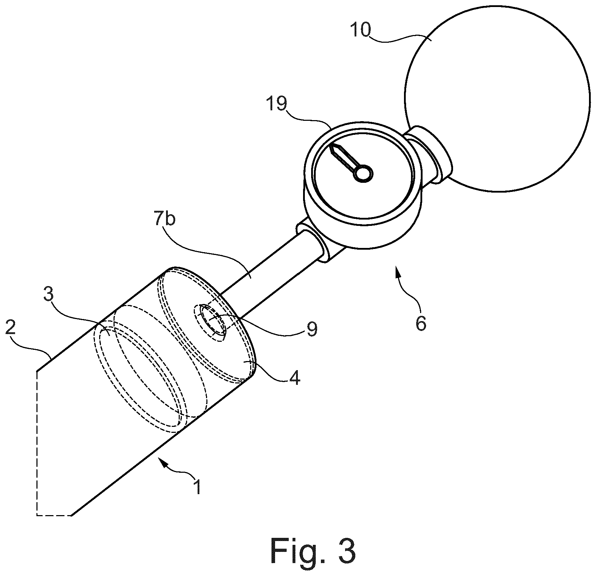

Fig. 3 shows an embodiment according to the invention comprising a push device 6 attached or fixed to a container 1.

A connection 7 is attached to an opening 9 at the first end 4 of the container 1. The connection 7 may comprise a soft and pliable pipe for air or other gas or liquid which is attached to the container 1 e.g. via a hub or socket provided at the first end 4 of the container 1. Alternatively, connection 7 comprising or being constituted of a pipe may be permanently attached to or fixed to the container 1. Whether a permanent or releasable attachment of the connection 7 to the container 1 is preferred may depend on whether the device is to be used multiple time or is for single use only, or whether the device is to be used in a hospital or at home by a single patient.

Beside the connection 7, the push device 6 also comprises a handle 8 in form of a hand pump ball 10 and a gauge 19. During operation, the user pumps, presses and releases the hand pump ball 10 thereby forcing air through the connection 7 and into the upper volume in the container 1 where the upper volume is the volume between the piston 3 and the first end 4 of the container. As the pressure in the upper volume increases, the piston 3 is pushed downwards towards the second end 5 of the container 1.

The device according to the invention may be used according to the following method:
a) Preparing a device according to the invention for use by placing the piston 3 of the device in a retracted position, i.e. a position closer to the first end 4 of the container 1 than to the second end 5 of the container 1. The position of the piston 3 - and the length of the container 1 - should allow for insertion of the prolapsed stoma in its full length.
b) The second end 5 of the container 1 is pushed against the abdominal wall around the prolapsed stoma, if the device comprises a connecting surface 13, this surface 13 is pushed towards the abdominal wall around the prolapsed stoma. By this operation, the prolapsed stoma is inserted into the container 1 through the opening in the second end 5 of the container 1.
c) If possible, fluid may be pumped into the container 1 to establish desired conditions around the prolapsed stoma.
d) The user then starts to push the piston 3 towards the second end 5 of the container 1. The pushing may be completely manual i.e. the user pushes a handle 8 which directs a rigid connection 7a in the longitudinal direction of the container 1, or the pushing may be done by increasing the fluid pressure above the piston 3 e.g. by pumping with a hand pump ball 10, or any other method.

The user may be the patient himself/herself as it is possible to use one hand to push the second end 5 of the container 1 towards the abdominal skin while the other hand is used for displacing the piston 3. However, the user may also be a doctor or a nurse or another medically educated person.

During operation of the pushing device, the prolapsed stoma will be forced back behind the abdominal skin.

e) The device is removed from the operational position, and the stoma may be secured in the position behind the abdominal wall e.g. with a positioning unit 14.

| **Ref. no.** | **Ref. name** |
|---|---|
| 1 | Container |
| 2 | Side walls |
| 3 | Piston |
| 4 | First end of container |
| 5 | Second end of container |
| 6 | Push device |
| 7a | Rigid connection |
| 7b | Conduit |
| 8 | Handle |
| 9 | Opening of first end of container |
| 10 | Pumping unit |
| 11 | First opening near second end |
| 12 | Second opening near second end |
| 13 | Connecting surface |
| 14 | Positioning unit |
| 15 | Sealing ring |
| 16 | Edge part of positioning unit |
| 17 | Vaulted central part of positioning unit |
| 18 | Opening of vaulted central part |
| 19 | Gauge |

## Claims

1. Device for repositioning a stoma prolapse, comprising
- a container (1) comprising side walls (2), a first end (4) and a second end (5) wherein the second end (5) comprises an opening,
**characterized in that** the device further comprises
- a piston (3) dimensioned to travel inside the container (1) from the first end (4) of the container (1) to the second end (5),
- a push device (6) configured to move the piston (3) from a first position to a second position close to or in contact with the second end (5).

2. A device according to claim 1, wherein the push device (6) comprises a rigid connection (7a) extending from the piston (3) to a handle (8) outside the container (1).

3. A device according to claim 1, wherein the push device (6) is connected to an opening (9) at the first end (4) of the container (1) which opening (9) via a conduit (7b) is connected to a pumping unit (10) pumping a fluid such as air.

4. A device according to any previous claim, wherein the container (1) comprises an opening (11) at or near the second end (5) of the container (1).

5. A device according to any previous claim, wherein the container (1) comprises at least two openings (11, 12) at or near the second end (5) of the container (1) where a first opening (11) may function as an inlet opening and a second opening (12) may function as an outlet opening.

6. A device according to any previous claim, wherein the device comprises a connecting surface (13) at the second end (5) configured to provide a liquid tight contact.

7. A device according to any previous claim, wherein the sidewalls (2) of the container (1) constitute a tube having a round or oval or elliptic or polygonal cross section, and the cross section of the tube (2) may vary in the longitudinal direction e.g. as in a truncated cone.

8. A device according to any previous claim, wherein the container (1) is made of a rigid, preferably watertight, material such as a polymer, laminated cardboard, according to a preferred embodiment the material is also transparent such as e.g. acrylic, pvc, polycarbonate or the like, also the material may be adapted for multiple use or for single use.

9. An assembly comprising a device according to one of the claims 1-8 and a unit (14) to be positioned at the stoma, wherein the unit (14) is placed inside the container (1).

10. An assembly according to claim 9, wherein the unit (14) comprises an edge part (16), a vaulted central part (17) which vaulted central part (17) comprises an opening (18).
